# EUROPEAN PATENT APPLICATION

(11) **EP 4 610 995 A1**
(43) Date of publication of application: **03.09.2025**
(21) Application number: 24160722.5
(22) Date of filing: 29.02.2024
(51) Int. Cl.: G16H 20/40, G16H 40/60

(54) **ARTIFICIAL INTELLIGENCE-BASED PLANNING AID FOR SPINAL SURGERY**

(71) Applicant: Spinem Robotics, 38400 Saint-Martin-D'Hères (FR)
(72) Inventor: MORVAN, Stéphane, 69260 CHARBONNIÈRES-LES-BAINS (FR)
(74) Representative: Germain Maureau

(57) **Abstract**

A device for assisting a surgeon in a preoperative planning of a surgical operation on a patient comprises a software unit using a predictive algorithm to produce predictions of surgical outcomes. The software unit is trained using learning data (401) comprising preoperative data (410), intraoperative data (420) and postoperative data (430) collected for a determined patient population during prior surgeries of same nature. The preoperative data (410) comprise data relating to a clinical profile of prior patient (400) and data relating to a surgical protocol that was implemented during the prior surgery. The intraoperative data (420) comprise recordings representative of trajectories as a function of time of the position and/or orientation during the prior surgery of an anatomical element concerned. The postoperative data (430) comprise data representative of surgical outcomes of the prior surgeries. The software unit produces predictions of surgical outcomes for the surgical operation concerned based on preoperative data associated with said surgical operation.

## Description

### BACKGROUND

### Technical Field

The present invention generally relates to methods and apparatuses for assisting surgeons in the planification of orthopaedic surgery.

It finds non-limiting applications in the field of spinal surgery, and concerns a device and a method for aiding the planning and/or execution of a surgical operation, for example the implantation of an intervertebral spacer or the insertion of pedicle screws, or spinal fusion.

### Related Art

The approaches described in this section could be pursued, but are not necessarily approaches that have been previously conceived or pursued. Therefore, unless otherwise indicated herein, the approaches described in this section are not prior art to the claims in this application and are not admitted as being prior art by inclusion in this section.

One type of spinal surgery focuses on restoring the intervertebral space, i.e., the space between two adjacent vertebrae in a patient's spine, by filling the space between the respective end plates of a lower and upper vertebra. This is also referred to as vertebral fusion in the jargon of the skilled person. This space is normally occupied by the intervertebral disc, whose function is to soften the tilting movements of one vertebra relative to the other. Degeneration of the intervertebral disc due to aging can render this space insufficient to allow local nerve structures to function properly. This is, at the very least, a source of pain for the patient that can evolve into loss of motor or sensory functions to the limbs.

Surgical intervention generally involves removing the malfunctioning intervertebral disc, and placing an implantable device between the two vertebrae concerned, known as a "spacer", "fusion cage" or "interbody cage". The pose of this implant is further secured with separate screws inserted into the upper and lower vertebrae and connecting rods. The implant restores a fixed vertical spacing between the two vertebrae and while subsequently eliminating all relative mobility with the screws and their connecting rods to allow the formation, over a 6-12 months period, of a bony bridge between the vertebra where the fusion is sought.

This intervertebral space is defined by two main parameters, namely the height of the intervertebral disc and the lordosis, which represents the angle formed by the respective vertebral plates of two adjacent vertebrae. Each level of the spine has a different lordosis and height from the others, and the most determining lordosis angle being those of the lumbar segments L4-L5 and L5-S1 which influence the overall spinal posture, and are also the sites of the most common surgical interventions.

This type of surgery is well known per se. It has been practiced for over four decades, particularly in the lumbar segment of the spine, but not exclusively. There is a wide variety of implants, surgical access techniques (also known as approach techniques), materials, surgical tools, and so on available to the surgeon to deliver the best care. During an operation and based in particular on the two aforementioned parameters and on his/her experience, the surgeon selects from a set range of implants (*i.e.*, fusion cages) the one best suited to the patient's physiology and pathology, in order to restore an intervertebral space that eliminates the patient's discomfort.

It is known, further, that surgeons can also be offered implants designed specifically for the patient and surgical procedure in question (so called "patient specific implants").

Despite global progress in intervertebral surgery, some surgical procedures do not produce good results. In particular, it is sometimes discovered during follow-up visits with patients 3, 6 or 9 months after surgery, that after some improvement following the operation, the patient is still experiencing pain. The patient's self assessment of his or her pain can be qualified by the score obtained on a questionnaire such as the SF-36, or any equivalent questionnaire. Such questionnaires contain a number of very general questions that cover, as broadly as possible, though by no means exhaustively, various possible manifestations of the success or failure of the surgery performed by assessing a patient's living condition after the surgery. Other, less subjective post-operative data may also be collected, for example, by means of medical imaging, including lordosis measurements and/or measurements of the height of the intervertebral space in question. In extreme cases of poor outcome, the doctor may even advise the patient to undergo further surgery, with all its inherent inconveniences and disadvantages that it entails, including another difficult recovery stage and the need for the patient to undergo post-operative rehabilitation, not to mention the cost to the healthcare system.

It appears that the choice of implant based on the determination of the two parameters explained above, which are the essential parameters supposed to have a direct influence on the success of the surgery performed, is the subject of much debate in the scientific literature. Some poor outcomes can be readily explained when a fusion cage was chosen to re-establish a certain height between the operated vertebrae, whereas in practice the space between said vertebrae obtained by placing this implant is less.

Thus, a "loss of correction" is frequently observed between the moment when the patient, lying on the operating table (ventrally or dorsally), receives the implant and the moment when the patient stands up and applies mechanical loading to the implant through his/her upper body weight and the associated muscle action. The loss of correction then manifests itself in the short to medium term (i.e., after a duration of more than 3 months and less than 24 months), by neurological disorders (pain, loss of various sensory functions) in the event of insufficient height restoration which can be attributed to either an unsuitable choice of implant height, or when the implant 'sinks' into the adjoined endplate vertebrae (termed subsidence).
In addition, in the longer term (*i.e.*, after 8 years), the suppression of mobility at the operated level has been observed to accelerate degeneration of adjacent spinal levels, typically located at the immediately adjacent intact level (in the cranial direction). Also, in the event of unsuitable choice of implant lordosis, the patient's posture is greatly altered in the upper segments which tries to compensate and reduce the amount of force required to maintain a comfortable free-standing posture. This compensation mechanism creates additional mechanical loading on the immediately adjacent (and therefore unoperated) anatomical levels, leading to accelerated aging of the latter with the apparition of associated neurological disorders, frequently referred to adjacent segment disease (ASD)

In the current state of the art, each surgeon integrates all this information indirectly, through his or her initial training, experience, continuing education, patient recruitment, evolution of practice, etc. In no specific order, the implant selected, the surgical approach and the surgeon's' surgical skills are the single most significant contributing factors on the surgical outcomes for a given patient. State-of-the-art solutions to this problem of poor outcome take a long time to achieve convergence, since the underlying biomechanical, physiological aspects are poorly understood at best in the context of a degenerated spine and, more so when it has been operated on. Improvement over time in the outcomes obtained by a particular surgeon may be subject to fluctuations as the surgeon changes equipment, hospital (and therefore possibly patient recruitment), etc.

### SUMMARY

The aim of the invention is to remedy, at least in part, the drawbacks of the prior art as set out in the above section. In order to achieve this result, the embodiments of the invention draw inspiration from predictive methods based on deep learning and artificial intelligence. The invention is based on the observation, indeed, that in the case of robot-assisted operations, carried out with the aid of either a robotic system and/or a freehand navigation system, a great deal of data is generated which remains unexploited beyond its initial production purpose.
More specifically, a first aspect of the present invention relates to a device for assisting a surgeon of interest in a preoperative planning and/or in the execution of a surgical operation on a given anatomical site of a patient of interest, comprising a software unit adapted to produce, during a planning stage, predictions of surgical outcomes for the surgical operation under consideration, based on a predictive algorithm using preoperative data associated with the surgical operation under consideration, wherein:
- the software unit is configured to be trained using learning data which comprise preoperative data, intraoperative data and postoperative data collected for a determined patient population during prior surgeries of same nature as said surgical operation under consideration which have been performed at the same anatomical site on respective prior patients of said patient population with the assistance of a localization system, of which, respectively:
   -- the preoperative data comprise, for each prior patient of said determined patient population:
      --- data relating to a clinical profile of said prior patient; and,
      --- data relating to a surgical protocol that was implemented during the prior surgery on said prior patient;
   -- the intraoperative data comprise recordings representative of trajectories as a function of time of the position and/or orientation during the prior surgery of at least one anatomical element, relative to said prior patient's anatomical site; and,
   -- the postoperative data comprise data representative of surgical outcomes of the prior surgeries for each prior patient of the determined patient population,
- the software unit is configured to produce predictions of surgical outcomes for the surgical operation under consideration based on the preoperative data associated with said surgical operation under consideration, which comprises:
   -- data relating to the clinical profile and/or patient reported outcomes of the patient of interest; and,
   -- data relating to a surgical protocol being considered by the surgeon of interest for the surgical operation under consideration.
A second aspect of the present invention relates to a method for assisting a surgeon of interest in a preoperative planning and/or in the execution of a surgical operation on a given anatomical site of a patient of interest, the method being implemented by a software unit and comprising the production, during a planning stage, of predictions of surgical outcomes for the surgical operation under consideration, based on a predictive algorithm using preoperative data associated with said surgical operation under consideration, wherein:
- in an unsupervised learning phase, the software unit is provided with an input dataset comprising learning data which includes preoperative data, intraoperative data and postoperative data associated with a plurality of prior surgeries of same nature as the surgical operation under consideration which have been performed at the same anatomical site on respective prior patients with the assistance of a localization system, of which, respectively:
   -- the preoperative data comprise:
      --- data relating to a clinical profile and/or patient reported outcomes of the associated prior patient; and,
      --- data relating to a surgical protocol that was planned and implemented during the associated prior surgery;
   -- the intraoperative data comprises recordings representative of trajectories as a function of time of the position and/or orientation during the prior surgery of at least one anatomical element operated on at the associated prior patient's anatomical site, relative to said prior patient's anatomical site; and,
   -- the postoperative data comprises data representative of surgical outcomes of the associated prior surgery,
- in a production phase, the software unit produces at least one prediction of surgical outcomes for the surgical operation under consideration based on the preoperative data associated with said surgical operation under consideration, which comprises:
   -- data relating to the clinical profile and/or patient reported outcomes of the patient of interest; and,
   -- data relating to a surgical protocol being considered for the surgical operation under consideration.

A third aspect of the present invention relates to a computer program product comprising one or more stored sequences of instructions that are accessible to a processor and which, when executed by the processor, cause the processor to carry out the steps of the method of the second aspect of the present invention.

Thanks to the above-mentioned features, the device and the method makes it possible to use previously collected data relating to previous surgeries performed on a number of previous patients, in order to considerably improve the reliability of the surgical outcomes obtained for a patient of interest. To this end, the algorithm assists the surgeon during the planning step of the surgery, by providing predictions of the level of correction that will actually be obtained based on the specifics of a surgical operation envisaged for the patient in question.

Use of the device and implementation of the method will bring benefits to patients, medical practitioners and the healthcare system as a whole. These include more effective pre-operative planning, more precise and efficient operations, improved decision-making and outcomes for patients, greater predictability for patients, more precise and less invasive surgical procedures, and reduced errors and risk of deviation during surgery.

In a direct manner, updating the predictive algorithm enables the surgeon, to:
- improve the efficacy of his/her interventions over time,
- maintain the efficacy of his/her interventions in the multiple healthcare centers where he/she operates, thanks to the updating of the predictive algorithm on the basis of data relating to interventions performed within several hospital sites, and possibly on several surgical platforms within the same hospital site. In one example, these data can be marked with dedicated information identifying the hospital site and, where applicable, the specific surgical platform concerned,
- detect and identify any deviations in its surgical outcomes, and address them by taking them into account for any new planned intervention,
- benefit from all the expertise of a more experienced surgeon by recovering, using, substituting (even temporarily) in whole or in part, the parameters of his/her personal predictive algorithm.

From the perspective of the manufacturer of the implants, the invention is also advantageous because it enables:
- to have an idealized model of the implant size distribution used by a surgeon, and to help him/her predict which implant references should be kept in stock in the healthcare facility vs. those classified as outliers which could only be delivered on special request,
- to use this idealized model to detect possible range extensions, i.e. intermediate or extreme sizes that do not otherwise exist in the marketed product line.

Further embodiments and advantages of the invention will become apparent from the description which follows, with reference to the accompanying drawings.

### BRIEF DESCRIPTION OF THE DRAWINGS

Embodiments of the present invention are illustrated by way of example, and not by way of limitation, in the figures of the accompanying drawings, in which:
- Figure 1 shows schematically frontal and lateral views of the whole skeleton of a human being, with an extracted representation of their spine;
- Figures 2A and 2B are a side view and a posterior view, respectively, of the lower lumber vertebrae of a patient's spine, illustrating the result of a surgical operation called "posterior lumbar fusion" performed with respect to lumbar vertebrae L3 and L4;
- Figure 3A is a block diagram illustrating the main elements of a surgical platform adapted for carrying out embodiments of the invention;
- Figure 3B is a schematic isometric perspective view of the surgical platform incorporating the device and suitable for implementing the method according to the proposed solution;
- Figure 3C is a schematic top view of a robot shown in Figure 3B, in operation over a patient laying ventrally on an operating table;
- Figure 3D is a schematic view showing details of the end of a robotic arm as shown in Figure 3B and Figure 3C;
- Figure 3E is a schematic view illustrating a localization system of the surgical platform of Figure 3B;
- Figure 3F is a schematic isometric perspective view illustrating an implant holder suitable for being used in the surgical platform of Figure 3B, with the implant assembled;
- Figure 4 is a schematic block diagram illustrating the generation of prior patient records suitable for being used as learning data for training the predictive algorithm according to embodiments;
- Figure 5 is a step diagram illustrating steps of an unsupervised machine learning scheme suitable for training the predictive algorithm with a dataset of learning data consisting of a set of prior patients' records as shown in Figure 4;
- Figure 6 is a schematic block diagram of software means adapted for training the predictive algorithm by implementing the unsupervised machine learning scheme as shown in Figure 5; and,
- Figure 7 is schematic block diagram illustrating a production phase, wherein the predictive algorithm according to embodiments is used by a surgeon for planning a new surgical operation for a patient of interest.

### DESCRIPTION OF PREFERRED EMBODIMENTS

The figures and the following description illustrate a specific exemplary embodiment of the invention. It will thus be appreciated that those skilled in the art will be able to devise various arrangements that, although not explicitly described or shown herein, embody the principles of the invention and are included within the scope of the invention. Furthermore, any examples described herein are intended to aid in understanding the principles of the invention, and are to be construed as being without limitation to such specifically recited examples and conditions. As a result, the invention is not limited to the specific embodiments or examples described below, but by the claims and their equivalents.

In the figures of the accompanying drawings, like reference numerals refer to similar elements, unless stated otherwise. In addition, and unless specifically indicated to the contrary, the disclosures contained in the entire description can be applied analogously to the same parts with the same reference signs or the same component identifiers.

In the following description, various embodiments will be explained with reference to the non-limiting example of planning spinal surgery and, more specifically, spinal fusion surgery, which generally consists of implanting an intervertebral fusion cage in a patient's spine and using pedicle screws and rod to secure it. The person skilled in the art will appreciate, however, that the teachings of the present description can be applied to other types of orthopedic surgery, for example knee surgery, ankle surgery, and any other surgery performed on bony joints such as the hip, the shoulder and the wrist, for example. Furthermore, the scope of the embodiments described herein and illustrated by the appended drawings in the context of spinal surgery may even encompass any other type of human or animal surgery that will similarly benefit from the advantages of the proposed device and method for planning a surgical operation.

The following terms used throughout the description are to be interpreted, such being the case, based on the definitions provided in what follows.

The term "intervertebral disc" refers to a soft tissue forming a solid but deformable layer interposed between the vertebral bodies, the main effect of which is to separate the two vertebral bodies and provide limited amount of viscoelastic shock absorption and mobility between the adjoined vertebral bodies. The space between the vertebral bodies allows the upper vertebra to tilt forward or backward without its distal edge coming into contact with the vertebral body of the lower vertebra.

Intervertebral space" or "interbody space" refers to the space between two adjacent vertebrae that is normally occupied by the intervertebral disc, and is therefore also known as the "disc space". The intervertebral space is defined by at least the following parameters:
- the height (in millimeters) between the upper surface of the lower vertebral endplate and the lower surface of the upper vertebral endplate; and,
- the lordosis (in degrees) which represents the dihedral angle formed by the respective vertebral endplates of two adjacent vertebrae. Each stage of the spine is likely to have a different lordosis from the others. The most important angles are those of the lumbar segments L4-L5 and L5-S1, which are also the sites of the most common surgical procedures; and,
- the cross-sectional area (in square millimeters) of the vertebral endplates in the transverse plane, i.e., the plane transverse to the axis of the spine. This parameter is often reduced either as an ellipsis or as its enclosing rectangle defined by its width (from the patient's left to the patient's right) and the depth (from the patient's belly to the patient's back) of the vertebral endplates, these measurements each being expressed in millimeters.

By "fusion cage" or "interbody cage", it is meant an implantable intervertebral spacer, which is adapted to take place in the intervertebral or disc space, *i.e.,* the space between a lower and an upper operated vertebra, and to achieve fusion by completely immobilizing said operated vertebra. The material constituting such a fusion cage can be stainless steel-based, titanium-based, or based on a PEEK polymer (where PEEK stands for "Polyether-Ether-Ketone"), which is a radiolucent polymer offering excellent long-term biocompatibility. This fusion cage can further be designed to embed osteoconductive or osteoinductive materials such as bone substitutes, autologous grafts or bone morphogenetic proteins.

A fusion cage is implanted using a surgical approach selected for insertion according to vertebral type, pathology, and patient condition. The surgical approach technique chosen will have an influence on the cross-section and stability of the implant, which helps mitigate cage migration. There are four main surgical approaches:
- the anterior approach: the fusion cage is inserted via the anterior part of the vertebra. This is known as the ALIF approach (Anterior Lumbar Interbody Fusion) where large implants (with very large contacting surfaces, and thus reduced subsidence risks) are used to provide very good support to the anterior column. Inserting these implants in the disc space requires moving key vascular structures out of the way, and is therefore a fairly challenging approach since the risk of patient death is increased;
- the posterior approach: the fusion cage is inserted via the back of the vertebra, through an off-centered median superficial incision. This is known as the PLIF approach (Posterior Lumbar Interbody Fusion), these compact implants, with very small contacting surfaces and thus, high subsidence risks, can only be inserted in the disk space by shifting major neural structures temporarily out of the way. These implants were designed to be used in pairs (requiring two incisions per level) to increase their anterior column supporting abilities, but viable usage as single instances (and thus, single incisions) have also emerged;
- the transforaminal approach: This is known as the TLlF approach (Transforaminal Lumbar Interbody Fusion). This procedure enables the surgeon to insert the implant into the disc space without having to mobilize any neural structures through a single paramedial incision. This not only reduces the risk of injury compared to a PLIF approach, but also provides better anterior column support as the cage is positioned perpendicular to the sagittal plane;
- the lateral approaches: DLIF (Direct Lateral Interbody Fusion) and XLIF (Extreme Lateral Interbody Fusion), are somewhat similar techniques that are using large implants with excellent anterior column supporting abilities (thus, low risks of subsidence) that are inserted into the disc space through natural passages free from major vascular or neural structures.

Whichever technique is used for implant insertion, the cages are further stabilized in their position using pedicle screws and rods, that provide posterior column support until bony fusion takes place. Those screws are always inserted through supplementary incisions and through the posterior access of the spine, making techniques that are using anterior, direct lateral or extreme lateral challenging since the patient must be operated through two distinct approaches. Surgeon may thus elect for a single surgery / single lateral patient position or single surgery / dual patient position (patient repositioning is done during surgery), or dual surgeries (typically a week apart) with different approaches.

Preoperative, intraoperative and postoperative data refers to data collected before, during or after, respectively, surgeries of the same nature as the surgical operation under consideration, namely the surgical operation to be planned. In particular, the term "intraoperative" is used in what follows to designate steps of the operative process that are implemented in the operating room, when anatomical elements of the patient and/or surgical instruments for the operation have been equipped with position and orientation trackers, and data is collected in that context. In the present specification, the words "position and orientation trackers" and "trackers" are used without any distinction. In the present case, embodiments use intraoperative data collected on the occasion of prior surgery identical or similar to the surgical operation under consideration, which has been performed previously on one or more other patients. Preferably, the prior surgery is a surgery performed on a group of prior patients operated by the same surgeon as the one who is to perform the surgical operation under consideration, or by any panel of surgeons including said surgeon. That way, the accuracy of the predictions expected from the implementation of the predictive algorithm upon planning the surgical operation at stake is enhanced.

The term "surgical procedure" encompasses all operative steps, medical devices and surgical products used, operative parameters selected, etc., which are carried out, used or set, respectively, during surgery. The procedures include the anaesthetics, the surgical approach, the type and/or reference of any robotic system and/or freehand navigation system used, and so on. The used devices include surgical instruments, like drills, cutters, rasps, bone rongeurs, curettes, shavers, scalpels, needles, etc. as well as implants and their holder, such as surgical screws, their associated connecting rods and locking nuts, etc. The used products comprise materials like graft bones, bone cement, etc. When used in reference to a scheduled surgical operation, these terms designate what results from the operative planning of the said operation.

A "Medical Outcome Study" (MOS) is an application of methods for monitoring the outcomes of medical care , and to compare the score obtained with that of a reference group of individuals. This source population may be healthy or suffering from the same pathology as the patient being assessed. The MOS can also be used to assess deterioration in a patient's state of health over a given period of time, by comparing scores obtained for the same patient, for example, from one year to the next.

MOS known as "Patient Reported Outcomes" (PROs) are health-related quality-of-life measurements used to evaluate and personalize treatment strategies. Taking them into account is a key factor in the success of medical and orthopaedic treatments. In practice, these measurements are derived from the patient's responses to a questionnaire such as, for example, the SF-36 (standing for "Short Form - 36"). SF36 is a questionnaire limited to 36 questions, which assesses a patient's state of health on a multidimensional, generic scale, i.e., independently of the patient's causal pathology, gender, age and treatment. As the SF-36 does not take into account a patient's age or gender, it can be offered to any patient. Further examples of PRO questionnaires include the Scoliosis Research Society-22r (SRS-22r) questionnaire, the Patient-Reported Outcomes Measurement Information System (PROMIS) questionnaire, etc. These questionnaires are completed before and after surgery. They are all equivalent, but not identical. A surgeon will generally choose a specific questionnaire according to his/her preference, specialty and training and as a metric to measure the efficacy of his/her work.

In what follows, "pelvic parameters" are patient parameters that characterize the spinal posture. They include the sacral slope, i.e., the angle formed between the upper plateau of the S1 vertebra and the horizontal (generally between 35° and 45°), and the pelvic version, i.e., the angle formed between the line joining the center of the femoral heads and the middle of the upper vertebral plateau of the S1 vertebra, on the one hand, and the vertical, on the other.

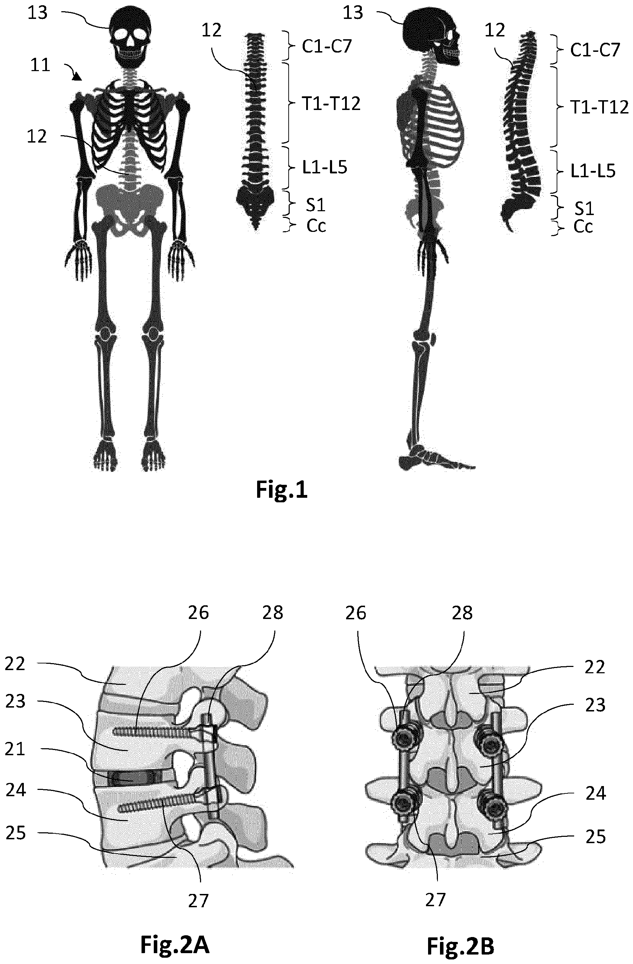

Referring to **Figure 1****,** there is shown therein schematic views of the whole skeleton 11 of a human being, with an extracted representation of their spine 12. More precisely, the left part of Figure 1 shows a front view, and the right part thereof shows a side view (from left) of said skeleton 11 and said spine 12. The human spine is a stack of bones called vertebrae joined by soft discs. It is made up of twenty-four vertebrae (or thirty-three if the fused sacro-coccygeal vertebrae are included). More specifically, the human spine comprises, from top to bottom and in this order, seven cervical vertebrae noted C1 to C7 (the first cervical vertebra C1, which is the highest, is also called the "atlas"), then twelve thoracic vertebrae noted T1 to T12, followed by five lumbar vertebrae noted L1 to L5. These vertebrae are followed downwards by the sacrum (S1) and the coccyx (Cc). The sacrum is made up of five fused sacral vertebrae, and the coccyx is made up of fourfused coccygeal vertebrae. The spine is curved in the median sagittal plane. It has a single primary curve (concave forwards), also known as kyphosis, in the thoracic, and two secondary curves (concave backwards) known as lordosis in the cervical and lumbar spines.

Each vertebra articulates with the vertebra above it and with the vertebra below it, except for the atlas, which articulates upwards with the occipital condyles of skull 13, and the coccygeal vertebrae at the very bottom of the spine. The vertebrae are joined by three joints, namely:
- the intervertebral disc, which unites the plates of two adjacent vertebrae; and,
- the two posterior inter-apophyseal joints or facet joints, where the upper articular processes of one upper vertebra articulate with the lower articular processes of the lower adjacent vertebra,
and stability is ensured by organic elements (not shown) including:
- ligaments;
- a complex array of spinal muscles that antagonize movement or stabilize the head; and,
- posterior apophyseal-joint or facet-joint capsules.

**Figure 2A** and **Figure 2B** illustrate schematically, in a side view and in a posterior view, respectively, the outcome of a surgical operation called "posterior lumbar arthrodesis". This is a surgical operation to hold vertebrae together, performed from the posterior side of the patient's back. In this example, the two vertebrae concerned are lumbar vertebrae L3 and L4. Posterior access is often preferred in the treatment of lumbar spinal canal stenosis, lumbar disc herniation, and lumbar degenerative spondylolisthesis. In Figure 2A and Figure 2B, four vertebrae 22, 23 24 and 25 are shown, which, for illustration purposes, are lumbar vertebrae L2, L3, L4 and L5, respectively.

When performing fusion at the level L3 and L4, the surgery usually begins by removing the intervertebral disc and ligamentum flavum between the lower vertebra 24 and upper vertebra 23. Next, the intervertebral disc is replaced with a bone graft, or an interbody fusion device such as a spacer 21 (as shown), which is inserted in place of said disc, to restore disc height and endplate orientation and improve the overall posture of the spine. Additional fixation devices including some type of metal screws and rods are then implanted in the vertebral bodies to provide further stabilization and immobilization of the segment. These implantable devices are designed to maintain the vertebrae 23 and 24 together while spine fusion occurs, typically over the course of 6 to 18 months. In the example as shown, two pairs of surgical screws 26 and 27, known as pedicle screws, are inserted within the vertebral body of each vertebrae, and secured together with two reinforcement rods 28 on either side. The role of this scaffold is to provide temporary support in a suitable configuration of the spine while a bony bridge occurs between L3 and L4.

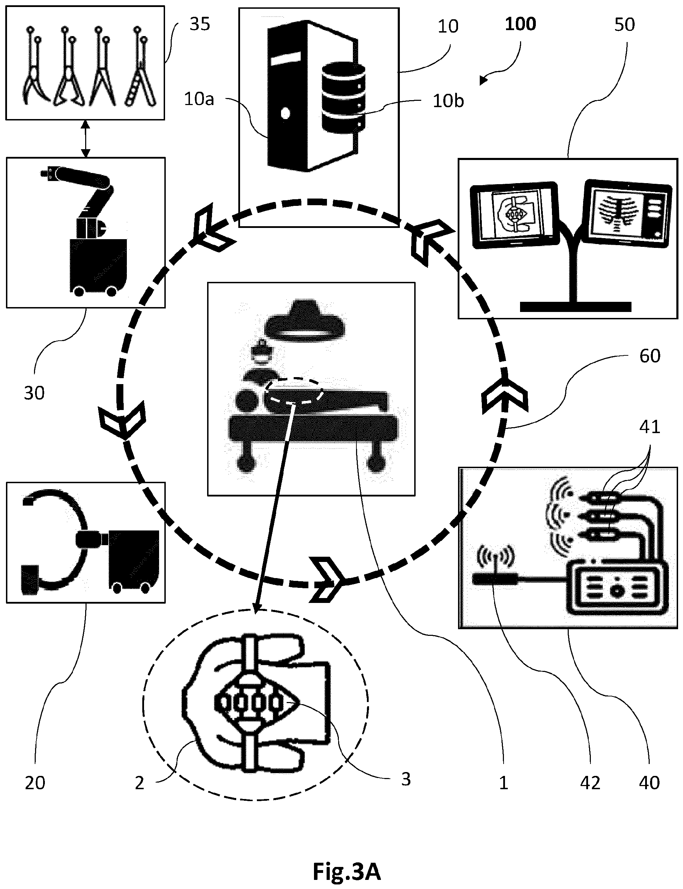

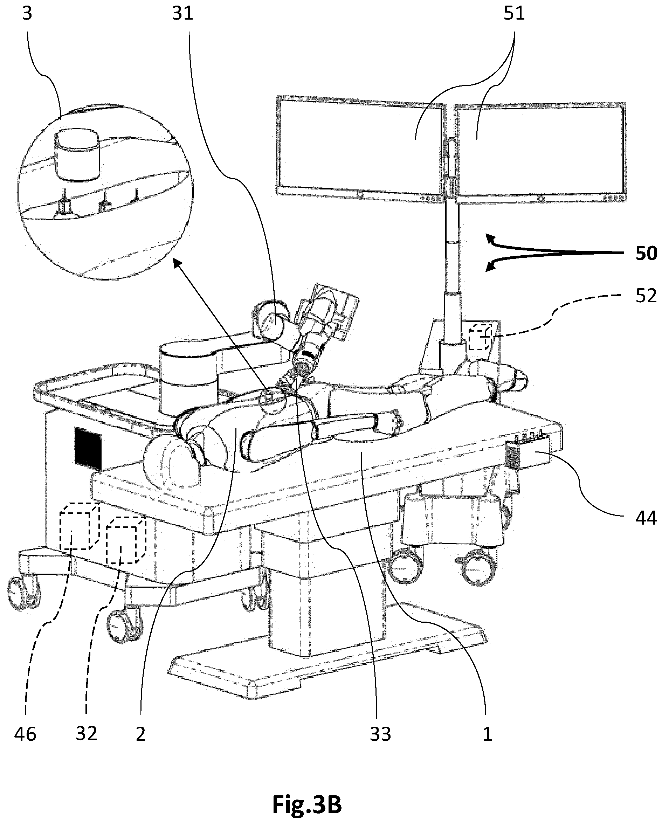

With reference to the block diagram in **Figure 3A****,** a robot-assisted surgical platform 100 - also referred to as "surgical system 100" - according to embodiments of the invention is adapted for collecting intraoperative data associated to prior surgeries of the same kind as the surgical operation of interest to be planned, as well as for subsequently performing said operation of interest. In said figure, functional blocks represent the main functional systems of platform 100, which shall be broadly described in what follows, with further reference, as the case may be, to the more figurative views in Figures 3B to 3F.

The surgical platform 100 first comprises an operating table 1, which is at the heart of the platform since a patient 2 may lie (or be laid-down) onto said operating table in a given operative position, intended for the performance of the operation. In the example as shown with more details in **Figure 3B****,** the patient is lying supine on the operating table 1, namely in ventral position, during the operation. Such position is convenient for operating the patient's spine while accessing the vertebrae posteriorly. It will be appreciated that, depending in particular on the anatomical site of the patient's anatomy wherein the operation is to be carried out, and depending further on the surgical access techniques chosen for the surgical operation, the patient can also be lying dorsally, or laterally onto the operating table 1 during the operation.

A further key element of the surgical system 100 is a central management system, namely a computerized management system 10. As diagrammatically shown in Figure 3A, this computerized management system 10 comprises central computing means 10a with one or more processors, and central data storage means 10b for permanently storing preoperative data, intraoperative data, and preferably postoperative data as well, as discussed in the present description of embodiments. These central computing means 10a and/or central data storage means 10b may be located within the operating room, but usually they are arranged in memory racks in a computer room of the healthcare center concerned. Alternatively, they may be hosted at least partially in a remote datacenter, and/or be implemented in a distributed computer architecture, for example in a High Performance Computing (HPC) data center. Whatever the practical implementation, the computerized management system 10 is configured for operational data communication, with respect to patient related information as well as control and supervision information with each of the other systems of the surgical system 100 described herein. This data communication is carried out via an ad-hoc data communication network 60, such as a dedicated local area network (LAN). Of course, encryption means are provided to preserve data integrity and confidentiality, in particular the confidentiality of patient records as required by applicable legislation.

The one with ordinary skills in the art will appreciate that the computerized management system 10 may be further configured to interact with at least one local computer, dedicated to performing specific computing tasks. Tasks performed by such local computer(s) will be discussed in more details in what follows, as they may be provided and configured:
- to process or at least pre-process image data acquired by an imaging system of the platform (see below);
- to implement algorithms described below in order to perform intraoperative data acquisition according to embodiments of the invention;
- to process localization data received from a localization system (see below); and,
- to assist the surgeon in performing the surgical operation, primarily by implementing near real-time visual representation of the instruments and implants in the patient's anatomy as they are either manipulated by the surgeon (hands-free navigation) or guided by a surgical robotic system.

The surgical platform may further comprise an imaging system 20 adapted to capture at least one image of the patient anatomy, more specifically of the patient's anatomical site 3 which is concerned by the surgical operation considered. The imaging system 20 can be, for instance, an X-ray two-dimensional (2D) imaging system and may comprise a CBCT-type X-ray medical imaging system, where CBCT stands for "cone beam computed tomography". Imaging system 20 will not be further described within the present description.

The person skilled in the art will appreciate that collecting such intraoperative data as those foreseen according to implementations of the proposed method, is rendered feasible by performing the surgical operations concerned while using the localization system that is using the means to localize (or track) instruments and anatomies in space to generate said localization data. Indeed, localization data associated to the patient's anatomical elements, to implantable device(s) and/or surgical instrument(s) involved in the surgical operation are intrinsically generated by that kind of system. Embodiments of the proposed method make a novel, and supplemental use of these localization data which are readily generated during such navigated surgery.

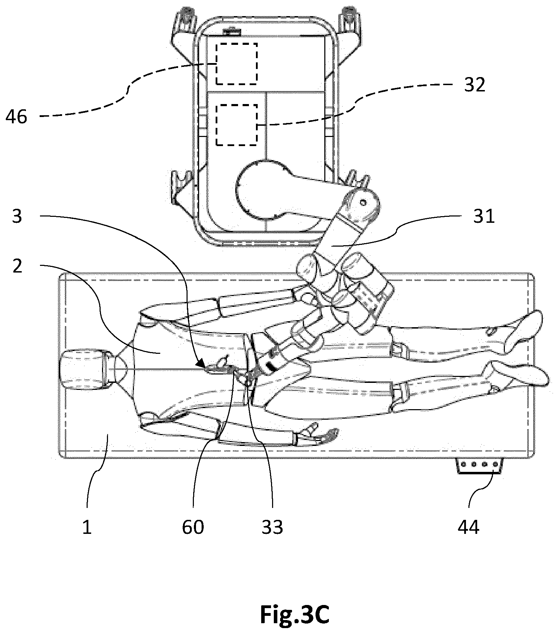

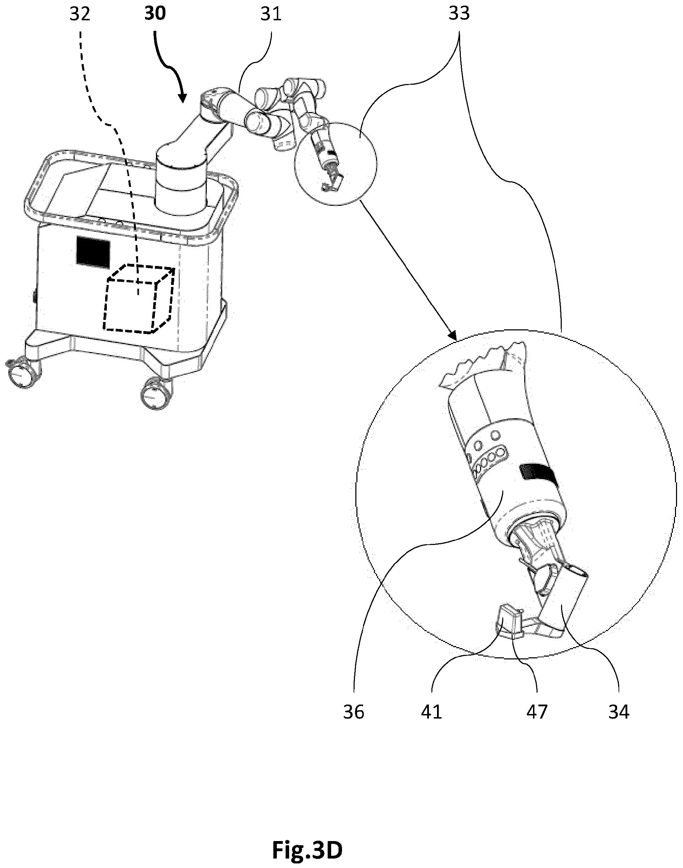

In some embodiments, the platform may comprise a surgical collaborative robot 30, which shall now be described with reference to **Figure 3B****,** **Figure 3C** and **Figure 3D****.** Mainly, the surgical robot 30 comprises an articulated robotic arm 31, which operates under control and/or supervision of an associated station 32. As shown in Figure 3B and Figure 3C, the articulated robotic arm 31 is located close to the operating table 1 and the patient 2 during surgery. Robotic arm 31 features for instance comprise a number of independent motorized axes, a particular mode, such as an automatic mode where the robotic arm is active or such as a collaborative mode wherein the robotic arm is directed by the surgeon, for instance through a control device - not shown on the figures. The articulated robotic arm 31 is equipped with an end-effector 33 described below with reference to figure 3D. The end-effector can be a passive end-effector comprising a surgical guide through which a surgical instrument can be inserted or the end-effector can be an active end-effector comprising an active surgical instrument such as an oscillating saw, a burr or a screwdriver for instance.

In some embodiments, the surgical platform 100 is a freehand navigation system. Such a freehand navigation system differs from the embodiment described above in that the surgical platform is deprived of articulated robotic arm. Thus, in such a freehand navigation system, the surgeon manipulates the surgical instrument by himself and only uses his/her knowledge and know-how to orient and guide a surgical instrument so as to position it or the attached implant at the anatomical site depending on the type of surgical operation under consideration. As mentioned above, the surgical platform further comprises a localization system 40 configured to track the trajectory and orientations of the surgical instrument and of the different anatomical elements of the anatomical site of interest, and consequently, if needs be, of the implant held by such surgical instrument and implanted in such anatomical site of interest where necessary. The localization system thus comprises, at least, position and orientation tracker(s) attached, directly or indirectly, to the anatomical site of interest, to the surgical instrument and/or to the implant.

The localization system comprises at least a tracker attached to the surgical instrument either manipulated by the surgeon or held by the articulated robotic arm. When the surgical platform comprises the articulated robotic arm 31, the localisation system 40 can additionally comprise, at least, a tracker attached to the end-effector of such articulated robotic arm. As detailed below, any configuration can be contemplated as long as the localization system permits to retrieve localization data related to the final pose of the implant with respect to the anatomical site and/or localization data related to different anatomical elements of the anatomical site with respect to one another. The localization system will be described with more details below. The control and/or supervision station 32 is implemented by a local computer, and is configured for acquiring localization data provided by the localization system. As mentioned, the localization data relates to the relative position of, at least, one surgical instrument with respect to the anatomical site of interest as well as to the relative position of different anatomical elements of the anatomical site, such as adjacent vertebrae, with respect to one another.

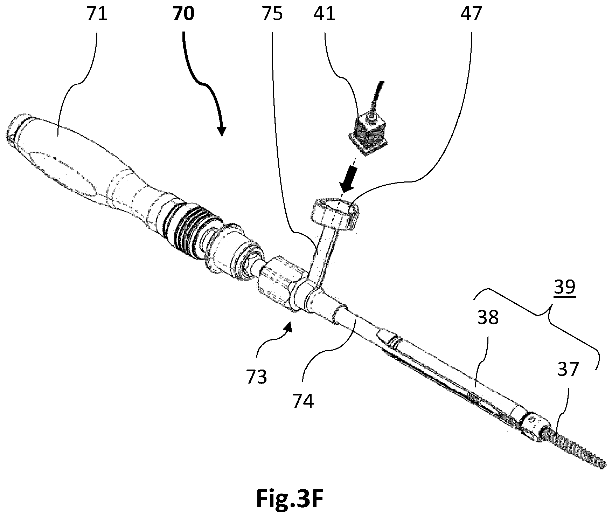

As shown schematically in Figure 3A, indeed, the surgical system 100 further comprises a set of robotic surgical instruments 35 associated with the surgical robot 30. With reference more specifically to **Figure 3D****,** the surgical instruments 35 are adapted to be inserted through a surgical guide 34 secured to the end-effector 33 of the robotic arm 31. The end-effector 33 may be located at the distal end of robotic arm 31. In the example illustrated in the detailed section of Figure 3D, the end-effector 33 may be equipped with an actuation control device 36, for example in the form of a ring as illustrated, having buttons configured to receive control commands from the surgeon during the operation, for example displacement commands to control the movements of the robotic arm 31. According to the illustrated embodiment, the end-effector 33 comprises a surgical guide 34 adapted to support a surgical instrument holder 70, as illustrated in Figure 3F, for example, which shall be discussed later in the present description. In other cases, the end-effector 33 may be adapted to directly accommodate the surgical instrument.

As mentioned above, the surgical platform comprises a localization system 40 (as shown on Figure 3A), preferably an electromagnetic localization system.

An electromagnetic localization system generally consists of a transmitting device, also called transmitter or field generator, consisting of one or more transmitting coils rigidly linked to each other, and of at least one receiving device, called receiver, consisting of one or more receiving coils rigidly linked to each other. As well known in the art, the joint analysis and modelling of the magnetic fields emitted by the transmitting coils and the fields measured by the receiving coils makes it possible to determine the position and/or the orientation of the receiving device with respect to the transmitting device. Consequently, the respective position and/or orientation of objects linked to each of the receiving device and of the transmitting device can be determined as long as the geometry of the links between said transmitting or receiving devices and the objects to be tracked are known. When a voltage is imposed on the terminals of a transmitting coil, an electric current flows in the transmitting coil which then generates a magnetic field proportional to the current flowing through it and the shape of which depends on the characteristics of the coil (orientation, magnetic moment, shape ...). Typically, a transmitter comprises three coils and a receiver comprises three coils. For instance, the transmitter can comprise larger coils than the receiver. Therefore, nine measurements are obtained, and the six independent degrees of freedom of the position and orientation of a transform matrix between the receiver reference system and the transmitter reference system can be determined, for instance by least squares optimization methods. In many systems, it is possible to use one transmitter and several receivers, in order to determine the relative positions and orientations of these receivers with respect to each other. For example, one receiver can be attached to a bone, and another one to the tip of an instrument, so that the relative pose of the bone with respect to the tip of the instrument can be determined. Electromagnetic localization devices are well known in the literature, as for example in the document "Magnetic Position and Orientation Tracking System" by Frederick H. Raab et al. published in IEEE Transactions on Aerospace and Electronic Systems VOL. AES-15, No. 5 September 1979, or the document "La localisation spatiale d'outils chirurgicaux par systèmes électromagnétiques alternatifs. Applications et domaines de validité des modérations numériques" by Jeffrey Paille - Thesis, Université Joseph-Fourier - Grenoble I, 2004. HAL Id : tel-00006125, version 1.

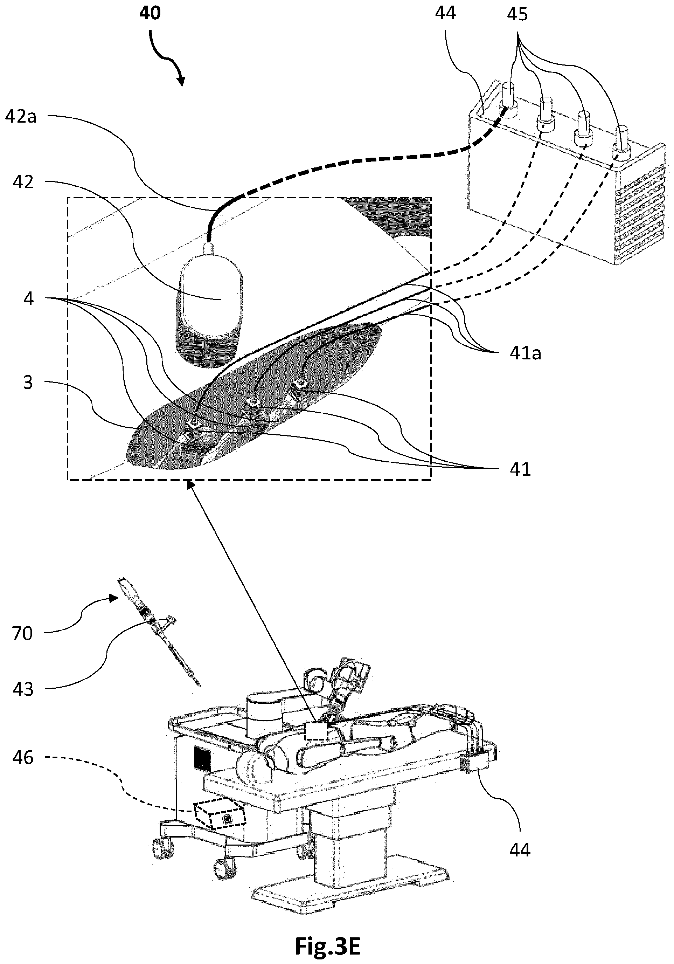

In exemplary embodiments of the localization system 40 of the surgical platform, a plurality of receivers 41 (also named electromagnetic trackers or simply trackers) and at least one transmitter 42 are provided, as shown in **Figure 3E****.** Said trackers 41 are adapted for being temporarily attached to anatomical elements 4 of the patient's anatomy, such as the spinous processes in the case of spine surgery. They can also be attached to surgical instruments or implantable devices, respectively directly, or indirectly that is to say, for instance to an instrument holder 70 as shown in Figure 3G, which holds a pedicle screw 39 consisting of its bone screw 37 and its articulated head 38. In embodiments as shown in Figure 3D, a tracker 41 can also be securely arranged at the distal end of the robotic arm 31 of the robot 30, for instance at the end-effector 33, according to a known geometry with respect to the surgical guide 34. Tracker 41 of Figure 3D is adapted to cooperate with the transmitter 42 of the localization system 40 in order to track the actual position and orientation in space of the end-effector 33 of the robotic arm 31, and therefore of the surgical instrument and/or implant it carries.

According to the illustrated embodiment a tracker is securely attached to the surgical instrument holder 70. The implant is attached to such surgical instrument holder 70 according to a linkage 75 of known geometry. The localization system is advantageously configured to track the surgical instrument holder 70 until the implant is detached of said surgical instrument holder. Thus, the last recorded pose of the surgical instrument holder and of the anatomical site of interest are used together with the known geometry between the implant and the surgical instrument holder to compute the pose of the implant with respect to the anatomical site in which it was implanted.

Further, the one with ordinary skills in the art will appreciate that elements 41 and elements 42 can be functionally interchanged, i.e., elements 41 can be transmitters while elements 42 are receivers. In addition, these two types of elements can be just one type of element, configurable as a receiver or transmitter (they are then called transducers). In this case, they can be configured either as transmitters or receivers, depending on the specific requirements of each surgical operation.

Referring back to Figure 3E, electromagnetic trackers 41 are adapted for being attached to respective anatomical elements of the patient, such as its vertebrae concerned by the surgery in the present example. In this example, three trackers 41 are securely but non-permanently attached (e.g., using surgical screws, glue or bone-anchoring means) to each one of the vertebrae 31: the operated vertebra, the lower vertebra and the upper vertebra respectively, consisting of two segments of interest. In operation, the transmitter module 42 is placed close to the anatomical site 3 of interest. In the example as shown in Figure 3F, the transmitter module 42 is adapted to be placed on the patient's skin, here on his/her back, close to the operated anatomical site 3. The skilled person will appreciate that the distance between the trackers 41 and the transmitter/receiver module 42 is a compromise between the operational range of the electromagnetic field generated by the transmitter/receiver module 42, on the one hand, and the required free space that must be available in the vicinity of the anatomical site 3 of interest to allow freedom of movement of the surgical instruments. In practice, the maximum distance between the transmitter module 42 and the receiver trackers 41 depends on the transmission power of said module 42 and the reception sensitivity of said receivers 41. The electromagnetic link can be dimensioned so that, for example, the transmission power is sufficiently high that the transmitter module 42 can be placed at any desired distance from the operating table 1, so as not to interfere with any surgical procedures. In the illustrated example, the trackers 41 and the transmitter module 42 are connected by wires 41a and 42a, respectively, to a localization data processing device 44. For example, said localization data processing device 44may be included in, or be a peripheral device of a local computer 46 of the surgical platform 100, which manages the localization function of said platform. As further shown in Figures 3B, 3C and 3F, the data processing device may be arranged at some point close to the operating table 1, for instance attached to a corner of said table as shown. Cables 41a and 42a may indifferently be manufactured from copper conductors or fiber optics, indifferently carrying analog or digital signals.

In preferred embodiments, each of the electromagnetic trackers 41 is adapted to provide data representative of the position and/or orientation in space of the concerned anatomical element, surgical instrument, implantable device or any piece of equipment to which it is rigidly attached. Stated otherwise, each tracker 41 is adapted for generating three position coordinates, and three angular coordinates representative of the position and orientation, respectively, in the three-axis coordinate system of the emitter 42 from which it receives the magnetic field. In addition, the processing of this data by the localization system 40 generates at least one confidence indicator, which indicates the overall accuracy of the system at the time the data was generated. This/these additional indicator(s) advantageously form an integral part of the data collected.

Electromagnetic trackers have the advantage of being easy to use, highly accurate, small in size and inexpensive. However, it will be appreciated that electromagnetic trackers represent only one example, and that the tracking devices can use various other tracking technologies, such as optical, inertial and/or ultrasonic sensing. This innovative tracking system used in combination with a surgical robot or a navigation system allows minimally invasive orthopaedic surgery. Unlike optical tracking, indeed, the electromagnetic tracking system does not have line of sight issues. It is compact enough to be attached to individual anatomical segments such as bones (vertebrae, ilium, sacrum, etc). Surgical landmarks are thus faithfully positioned and oriented in space and time throughout the duration of the surgery.

With reference now to **Figure 3F****,** an instrument holder 70 is illustrated, holding an implantable device such as a pedicle screw to be implanted in the patient's anatomy. In the example shown in the figure, the instrument holder 70 holds an implant 39, which is securely yet removably held at one end of the instrument holder 70. In the example s shown, the implant 39 comprises a surgical screw 37 along with a protection tube 38 for protecting the patient's soft tissue during the operation. Said protection tube 38 is breakable from said surgical screw 37 once it has provided the desired functionality, typically at the end of the procedure.

The instrument holder 70 comprises a handle 71, a T-shaped central portion 73, and a socket 47 for connecting a tracker 41. The central portion 73 includes a longitudinally extending tube 74, configured to hold at one of its longitudinal ends a surgical instrument or any other medical device like implant 39 as shown in the example depicted in Figure 3F. The handle 71 can be removably attached at the other longitudinal end of tube 74. The central portion 73 further comprises a transverse linkage 75, which extends transversally from the longitudinal tube 74. The end of linkage 75 which is distant from said rod 74 holds the socket 47. An electromagnetic tracker 41 can be plugged into said socket 47. That way, while the surgical operation is in progress, any displacement (both in position and in orientation) of the surgical instrument or medical device, even while held by the instrument holder 70, will be navigated by the localization system 40. Thus, the trajectory of the surgical instrument or the medical device during surgery will be captured in real time, by storing associated position and orientation data at a 30 Hz or 60 Hz frequency, for instance.

Finally, the surgical platform 100 includes a user interface system 50 which is illustrated in Figure 3B. Said user interface system 50 is adapted to display information and receive inputs (commands, control data, etc.) from a user, typically the acting surgeon during the surgical operation. In the example shown in Figure 3B, the user interface system may comprise one or rather two (or more) anti-glare displays 51 designed to be operated by the surgeon under special purpose in sterile surgical fields. At least one of these can be a touch screen, which eliminates the need for the user to use a mouse and keyboard to interact with the interface. This screen is used to display graphically, for example, menus, virtual buttons and various virtual input devices and selection tools of this type. The user interface system 50 includes an interface data processing device 52. This device 52 may be implemented by software within a local computer, which is configured and programmed to access and manage patient records, for example to import and display them on the touch-sensitive screen by retrieving the corresponding data from database stored in the storage means 10b, to explore, modify and/or update them, etc., and more generally to enable the surgeon to interact with the useful data contained in these records during the operation. The user interface system 50 is further configured to access planning information relating to the scheduled surgical operation, including, for example, the characteristics of the implantable device(s), surgical instrument, surgical products, accessories, etc., in particular those which have been selected when planning the operation.

The other of the displays 51 may be a monitor display which is configured to display feedback information as part of the localization system of the surgical platform 100. It can display, e.g. in 3D or 2D representation, any information relating to the position and/or orientation of the anatomical elements being operated on (for example, bones such as vertebrae, etc.), and/or to the implantable device(s) being manipulated, and/or to the surgical instruments being used, based on the localization data provided by the localization system 40 in connection therewith. More generally, this screen is used to display in near real time, during the surgical operation, any reconstructed 3D image of the current state of the anatomical site 3 concerned, enriched by the precise location of the individual anatomical components and/or instrument navigation information at all stages of the surgical operation in progress. Thus, the surgeon is provided a realistic view of what is happening inside the patient's anatomy, as well as system status information (e.g., tracking status: good/bad, etc.).

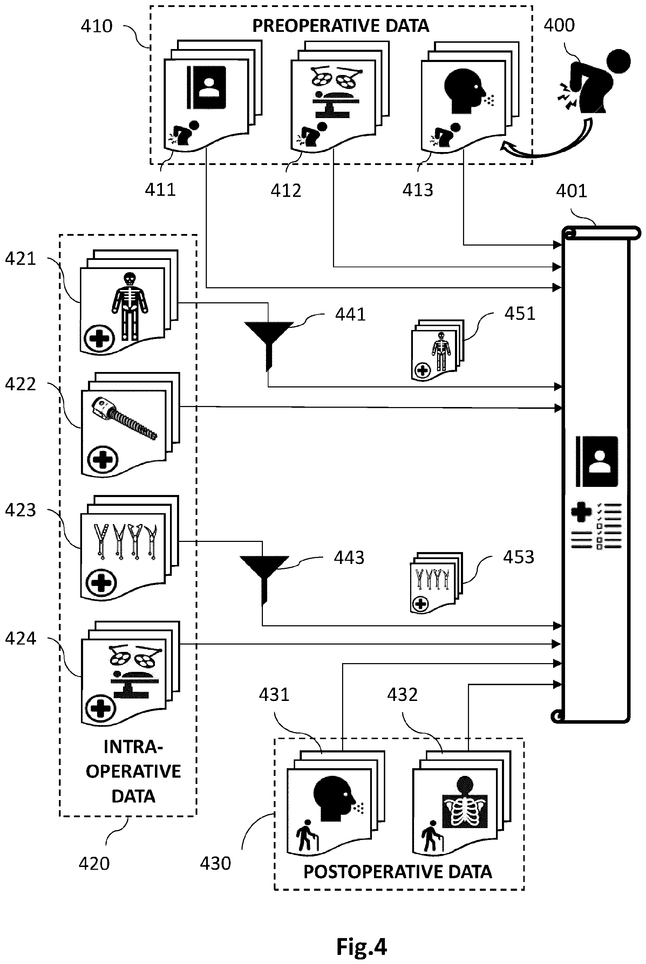

Turning now to **Figure 4****,** there is shown therein a block diagram illustrating the data collection process for building-up a record 400 of learning data associated to prior patients and to prior surgeries. In the context of the present description, an associated prior patient is a patient who has received surgery for the same kind of indication as the patient of interest whose surgical operation is at the planning stage. Thus, the learning data comprise data associated with a plurality of prior surgical prior surgeries of the same nature as said surgical operation under planning which have been performed at the same anatomical site on respective prior patients. In the present specification the words "surgical operation under planning" and "surgical operation under consideration" are used without any distinction. According to embodiments, the learning data comprise preoperative data 410, intraoperative data 420 and postoperative data 430 of said prior surgeries.

Preferably, the prior surgeries are surgeries that have been performed specifically by the surgeon of interest, *i.e.* the surgeon who is to operate on the patient of interest and who is therefore responsible for planning the surgical operation under consideration. Some prior surgeries may have been performed by a college of surgeons including said surgeon of interest, similar to a surgery learning environment. Thus, the predictions provided by the predictive algorithm according to the embodiments will be customized for said surgeon of interest.

In addition, said prior surgeries are surgeries which have been performed with a localization system, adapted to generate intraoperative data comprising records representative of trajectories, as a function of time of the position and/or orientation during surgery, of one or more anatomical elements operated on at the associated prior patient's anatomical site, relative to said prior patient's anatomical site. In some embodiments, the localization system was adapted to generate, further, time-dependent records representative of the use, within the associated prior patient's anatomical site, during the associated prior surgery, of one or more surgical instruments used by the surgeon of interest during at least one specific surgical step of said prior surgery. In some embodiments, these time-dependent records may reflect trajectories as a function of time and/or the dynamics of displacement of the instrument in space. It will be appreciated that the terms "intraoperative data" encompass a broad range of information with respect to surgeon's actions and, more generally actions taking place within the operating room. In some embodiments, for instance, one can track the moment of actuation of an electrosurgical drill used to prepare the initial contacting surface to prevent skyving, or to cut a hole in the pedicle, or to tap along that hole and prepare the screw passage. The advantage of the above-mentioned implementations is that it advantageously allows a greater capture of a surgeon's actions during all operating steps, and is therefore not limited to spatial position and orientation, but includes electrosurgical devices actuation (if applicable) as well.

According to embodiments, the preoperative data 410 associated to any one of the prior surgeries referred to in what precedes, comprises:
- preoperative data 411 relating to a clinical profile of the associated prior patients; and/or
- preoperative data 412 representative of Patient Reported Outcomes (PRO), stemming from the prior patients; and/or
- preoperative data 413 relating to a surgical protocol that was implemented during the associated prior surgeries.

The prior patient's clinical profile data 411 shall reflect some characteristics of the prior patient. These patient related data pertain to, e.g., demographic, comorbidities and/or past medical history information of the prior patient. They may include one or more of, for instance, the age (in years), the height (cm), the weight (kg), the Body Mass Index or BMI (kg/m²), whether or not the patient smokes, whether or not he/she is presently receiving another treatment (antidepressants, non-steroidal anti-inflammatory drugs etc...) or any other parameter or comorbidities deemed relevant by the practicing surgeon. In some embodiments, data relating to a clinical profile 411 of a prior patient 400 advantageously comprises categorical variables corresponding to personal information pertaining to said prior patient 400, respectively. For instance, this personal information can include one or more of gender (M=male, or F=female), the ethnical origins (ethnicity), the anatomical level of operated vertebrae within the patient's spine, frontal and/or profile X-ray images, Roussouly or Lenke classification, spino-pelvic configuration, patient score on a health questionnaire such as the SF-36, etc. Further data relating to characteristics of the prior patient which may prove relevant are, for instance, a Charlson Comorbidity Index or CCI that provides a weighted score of the patient's comorbidities which can be used to reflect short term and long-term outcomes such as function, hospital length of stay and mortality rates, a Frailty Score which reflects the patient's state of increased vulnerability to external stressors, previous Thoracic Lumbar Sacral Orthosis (TLSO) as the case may be, etc.

Additionally, said prior patient's clinical profile data 411 can further contain further patient related data, which may include data representative of measurements obtained from, e.g., standing X-Rays pictures. Such measurements can be made by a medical staff member (i.e., by a human), or may stem from automatic medical picture analysis performed by, *e.g.,* an Al-based software machine. Non limiting examples of such measurements include the following measurements, which are not exclusive and can be considered solely or in combination:
- Coronal and Sagittal Spinal Alignment primary measurements, such as: Lumbar Scoliosis (Cobb. Angle), Pelvic Incidence (PI), Pelvic Index- Lumbar Lordosis (PI-LL), T1 - Pelvic Angle (TPA), Thoracic Scoliosis, Thoracic Kyphosis (T2-T12), Lumbar Lordosis (L1-S1), Sagittal Vertical Axis (SVA), etc.
- Pelvic and Upper/ Lower Limbs secondary measurements, such as Pelvic Tilt (PT), Knee Angle (KA), shoulder imbalance, Sacro Femoral Angle (SFA), Pelvic Shift (P. Shift), etc.

The preoperative data 412 is representative of Patient Reported Outcomes (PRO). These may include scores to one or more standardized questionnaires filled-out by the patient himself or herself, before he or she had surgery, such as the Short Form Questionnaire (SF-36), or the Numerical Rating Scale (NRS) for evaluating back pain (NRS-Back) or leg pain (NRS-Leg), or a score (NRS20) for combined back and leg pain in range [0-20]. In a variant or in supplement, scores to other well-known questionnaires may be included in the preoperative data 412, such as one or more scales of the already mentioned PROM IS suite, e.g., PROM IS -Anxiety, PROM IS - Depression, PROM IS - Pain Interference, PROMIS - Physical Function; PROMIS - Social DSA (reflecting patient's satisfaction with participation in Discretionary Social Activities), PROMIS - Social Role, etc. Scores to one or more questionnaires of the SRS-22r suite which has readily be mentioned in what precedes may also be considered, in addition or as alternatives to the PROMIS questionnaire(s), for instance the SRS22 - Activity, SRS22 - Pain, SRS22 - Mental; SRS22 - Appearance, SRS22 - Total, etc. Still further MROs may include Oswestry Disability Index (ODI) which is a patient-completed questionnaire providing a subjective percentage score of level of disability in activities of daily living in those rehabilitating from low back pain. All of these are non-limiting examples only. Many other scores may be considered depending on the population and/or diseases concerned, such as, for instance, a Veterans RAND 12-item (VR-12) health survey like the VR12 MCS (Mental Component Summaries) or VR12 PCS (Physical Component Summaries), etc.

The preoperative data 413 may further comprise data relating to a surgical protocol that had been planned in advance of the prior surgery and, more specifically, was conducted during the associated prior surgery. These data may comprise, *inter alia,* details of the surgical intentions, such as the designated anatomical areas where the surgery was to be carried out (*e.g.* Lumbar vertebrae L1, L2, L3 etc...), the position and orientation of implants on or in the patient's anatomy as they were scheduled, the size and material of the implants that were considered for implantation during planning of the prior surgery, the specific access method (e.g., the surgical technique) intended to be used to insert the implants at their desired locations, etc. all of which being possibly represented as categorical data.

In some embodiments, preoperative data 413 relating to a surgical protocol that was implemented during the prior surgery concerned may include characteristics of one or more implantable devices (*e.g.* pedicle screws, intervertebral spacers or fusion cages, disc prosthesis, etc.) that has been implanted during said associated prior surgery. Such characteristics may include, in particular, the type, size, constitutive material, etc. of the surgical implant(s) that is (are) concerned. Considering the non-limiting example of a pedicle screw, such characteristics include, *inter alia,* the diameter, the type (for example monobloc, uniplanar, or polyaxial), the constitutive material (Titanium, Cobalt-Chromium or Carbon-reinforced PEEK), bone screw thread characteristics, and union rod characteristics such as diameter, constitutive material, etc. Characteristics of an intervertebral spacer can be the height, lordosis, cross-section, and/or constitutive material such as PEEK, machined titanium or 3D Printed titanium of said spacer as well as other any additional features like surface enhancements such as acid-etched or plasma sprayed titanium. Further, the presence or absence of a bone graft associated with the implanted spacer, such as a synthetic graft, an autograft graft and/or a xenograft graft may be additional features of interest as well as the use of osteo-inductors such as bone morphogenetic proteins. Such being the case, features of an implanted intervertebral disc prosthesis, can be its kinematics, constitution (material, shape), etc. all of which possibly being represented as categorical data.

Preoperative data 413 relating to the surgical protocol of the prior surgery may further comprise data relating to one or more implantable substances which have been implanted during said prior surgical operation. Non-limiting examples of such substances include, for instance, consolidating substances such as bone substitute or bone cement here too possibly being represented as categorical data.

In addition, the aforementioned preoperative data 413 may further include data relating to a surgical approach technique used to insert the above-mentioned implantable device(s) and/or implantable substance(s) in the patient's anatomy, which are collectively referred to as "implants" in the context of the present description. Reference is made, more specifically, to implants which have been inserted and implanted into the patient's anatomical site concerned, during the associated prior surgery. For example, features of the implantation technique that was employed to insert the implants during the prior surgery may include a surgical approach chosen to insert the implants, among a lateral approach (XLIF), a transforaminal approach (TLIF), an extra lateral or direct lateral anterior approach (DLIF), a posterior approach (PLIF) or an anterior approach (ALIF), or any other approach deemed suitable by the operating surgeon for example, all of which possibly being represented as categorical data.

Those of ordinary skill in the art will appreciate that the specific, yet non-limiting examples of preoperative data 410 listed above relate to the surgery taken as an example in the context of the present description, namely spinal fusion surgery. It goes without saying, however, that the embodiments of the invention encompass other spinal surgeries, as well as other orthopaedic surgeries such as ankle or knee restoration surgery, or hip or knee prosthesis implantation, for instance.

According to embodiments, the intraoperative data 420 associated to any one of the prior surgeries referred to in what precedes, comprise records 421 representative of trajectories as a function of time of the position and/or orientation during the surgery of at least one anatomical element operated on at the associated prior patient's anatomical site, relative to said prior patient's anatomical site. In the example of spinal fusion surgery, which is being considered here, the anatomical element concerned is the vertebra which is operated on.

For example, the aforementioned records may comprise time-dependent measurements of the intraoperative variation in position and/or orientation of the vertebra (or any other operated anatomical element, as the case may be) relative to the prior patient's anatomical site. The one with ordinary skills in the art will appreciate that such measurements can be provided by trackers 41 of the localization system 40 of the surgical platform 100 which has been presented above with reference, among other, to Figure 3E. Advantageously, such measurements are readily available, as they have been generated by the localization system which has been used to assist the surgeon in charge of the associated prior surgery. Stated otherwise, no additional hardware nor software means are to be provided to generate these measurements. Besides, the measurements are available, and are not generated specifically for the implementation of the proposed method but are instead a byproduct or a telemetry recording of the prior surgery as it was conducted by the operating surgeon.

In some embodiments, the intraoperative variation in position and orientation of the associated prior patient's operated vertebra (or other bone, or any other anatomical element operated on) is determined by translation values and rotation values, respectively, of said operated anatomical element, between the beginning and the end of the surgery. These values may be defined in a referential of the prior patient's anatomical site, for instance with respect to one of the lower and upper vertebrae.

In some embodiments, time-dependent trajectory records 421 of vertebral (or any other anatomical element of interest) positions and orientations in space, may be augmented with one or more of the following:
- Semantic labels resulting from surgeon or operating staff dictated oral or textual annotations nonspecific to the surgical step (e.g. general impressions or observations of a detail of the current step);
- Activity labels resulting from the precise operative step of the procedure as determined by the software state machine; and,
- State labels, e.g. the particulars of the surgical action, if any, performed on that particular vertebra, which can also be in the form of a dictated oral or textual label.

Without loss of generality, this data may also be recorded and stored as categorical data. In some embodiments, the intraoperative data 420 associated with the prior surgery further comprise translation values and/or rotation values 422, with respect to the associated prior patient's operated anatomical element, of one or more implants which have been implanted during the associated prior surgery. These translation values and rotation values reflect the changes in position and in orientation respectively, of the element being tracked. In case of spinal fusion surgery which is the non-limiting example considered for the purpose of the present description, wherein the anatomical element operated is a vertebra, the translation values and/or rotation values may be those of one or more implants such as pedicle screws, on the vertebral endplate of the operated vertebra. For instance, said translation values and rotation values may be defined in a referential of the prior patient's anatomical element onto which the implants have been implanted. In the above example, it may thus be a referential tied to the operated vertebra.

As was already explicated in the foregoing, in particular with reference to Figure 3F, the final position and orientation of the implant may be derived from the position and orientation, respectively, of the instrument holder 70 at the exact moment where the implant is released/disconnected from said holder and is left in the prior patient's anatomical site concerned. Stated otherwise, the translation values and/or rotation values of the implant with respect to the associated prior patient's operated anatomical element are derived from translation values and/or rotation values, respectively, of a surgical instrument equipped with a position and/or orientation tracker, such as tracker 41 in Figure 3F, which had been holding the implant at the time of placement and deposition of said implant in the prior patient's anatomy.

According to other embodiments, the intraoperative data 420 associated with the prior surgery may further comprise time-dependent recordings 423 which are representative of the use, during the associated prior surgery and relative to the associated prior patient anatomical site, of one or more surgical instruments used by the intervening surgeon during at least one specific surgical step of said prior surgery. These recordings can be used to define trajectories as a function of time and/or the dynamics of the surgical instrument's movement in space. These data reflect the handling of the instrument by a surgeon on the patient's anatomy during a specific stage of the surgery and is representative of the surgeon's actions during that particular stage of the surgery. When considered in combination with other intraoperative information as well as with preoperative and postoperative information, this information can be useful, for instance, in helping the surgeon to assess, after the surgery, the reasons for success or, on the contrary, the reasons for mixed outcomes or even failure of the surgery.

In some embodiments, time-dependent trajectory recordings 423 of the surgical instruments' positions and orientations in space, may be augmented with one or more of the following:
- Uncertainty factors in relation with the measurements, which can be derived from the localization system 40 (*e.g.,* confidence indicator...),
- Semantic labels resulting from surgeon or operating staff, *e.g.,* dictated oral or similar textual annotations specific to the surgical instrument (*e.g.,* general impressions or observations by the surgeon during use of the instrument),
- Activity labels indicative of the precise operative step of the procedure, *e.g.,* as determined by the application state machine,
- State labels, *e.g.* the particulars of the instrument, with details reflecting the conditions of use of the instrument during the surgery *e.g.:*
   ∘ in the case of a drill, for instance, whether the drill was powered at this specific timestep or not, and if so, at what settings;
   ∘ in the case of a screw driver, for instance the diameter, type and length of screw used etc.).

Still further intraoperative data 420 may additionally comprise, in some embodiments, standard operative variables 424. A non-exhaustive list of such variables includes, for instance, the surgery duration (in s), the patient's blood loss (in mL), the type of anaesthesia (which anaesthetic product has been used, and in what posology, etc.), the patient positioning during surgery (*e.g.,* ventral, lateral, dorsal), the suture type (*e.g.,* stitches, staples, etc.), whether the surgery was performed by a single operating or dual operating surgeons, whether there was any deviation from the initial plan (yes/no), use of blood transfusion (yes/no), the surgical approach (open or MIS, which stands for minimally invasive surgery), the incision sites and sizes, the drain usage duration, any perioperative antibiotic prophylaxis, etc. Again, and without loss of generality, this data may also be recorded and stored as categorical data wherever appropriate.

Finally, the postoperative data 430 comprise patient reported outcomes 431 (or PRO, standing for Patient Reported Outcomes) which are representative of surgical outcomes of the associated prior surgery. Such postoperative data are, indeed, representative of the surgical outcomes of the prior surgery. That form of outcomes reported by the associated prior patient are collected at one or several specific time intervals after the associated prior surgery, for example in the form of at least one score obtained from a health status questionnaire such as the SF-36 questionnaire. More generally, patient reported outcomes 431 are comparable in nature with the preoperative data 412 already detailed in the foregoing, namely they may include scores to one or more standardized questionnaires which have been presented in detail, with the noticeable exception that they are filled-out by the patient himself or herself, after he or she had surgery, either at home online or via an automated chat agent-like model.

The postoperative data 430 may further comprise further postoperative data 432 which are comparable to the further prior patient's data included in the clinical profile data 411 of said prior patient, as previously described. In practice, such postoperative data 432 may comprise data representative of measurements (of, *e.g.,* lordosis and/or intervertebral space height) obtained, for example, from radiographic images taken post-operatively. Non-limiting examples of such measurements include measurements such as those which have been listed in the foregoing with respect to the preoperative data of the prior patient's clinical profile 411 and which will not be repeated here. Those of ordinary skill will appreciate, however, that any comparison between the preoperative data of the prior patient's clinical profile 411, on the one hand, and the postoperative data 432, on the other hand, enables medical staff to appreciate the extent to which the surgery has altered the patient's situation from a clinical point of view, while not being sufficiently specific about the patient's exact condition, which is more adequately measured via the self-reported PROs 412 and 431 described earlier.

In embodiments as diagrammatically illustrated in Figure 4, the software unit of the device may comprise pre-processing modules 441 and 443, configured to reduce the dimensionality of recordings 421 and of recordings 423, respectively. It is recalled that these recordings are representative of the position and/or orientation values of the patient's operated anatomical element and representative of the use of the surgical instrument, respectively. In practice, they represent a significant amount of data. In some examples, recordings are generated at a frequency of 60 Hz or 120 Hz, meaning thousands of recordings over the standard duration of a commonly executed fusion procedure. Therefore, it is proposed that dimensionality reduction techniques be used to reduce the number of input data to retain the most meaningful information. Such techniques are embedded into pre-processing modules 441 and 443, which are configured to receive intraoperative data 421 and 423 in input, and to generate output data 451 and 453, respectively, of lower dimensionality, but with substantially the same meaningful content data.

Some dimensionality reduction algorithms known by the one skilled in the art include Principal Component Analysis, PCA. PCA transforms the original features contained in the high dimension input data into a lower-dimensional space while preserving the maximum amount of information. As a matter of example, one may consider a technique such as t-SNE (standing for "t-Distributed Stochastic Neighbour Embedding"), that visualizes high-dimensional data by reducing it to a lower-dimensional space while preserving local structure.

In variants, dimensionality-reduction modules 441 and 443 can be implemented as specifically designed and pre-trained auto-encoders.

Combination of such auto-encoders and processing based on Principal Component Analysis, PCA may also be considered, depending on the peculiars of any specific application.

With special reference to the intraoperative data 421 and recordings 423 of data representative of the use of the surgical instrument the one with ordinary skills in the art will appreciate that modules 441 and 443 are specifically designed to compute the signature of a surgeon's use of the instrument on the patient anatomy during a specific step of the surgery. This signature quantifies the action of the surgeon and assumes unicity, that-it-to-say that two nearly identical signatures represent two equally comparable (yet perhaps not strictly identical) actions. It also supports distance measurements such that two nearly identical signatures - as measured via conventional L0, L1, L2 normative distances, or Cosine similarity - represent nearly identical surgeon actions. This signature may then further be compared to the signature amongst other patients, which shall help appreciate consistency of a surgeon's actions during each operating step.

To summarize, preoperative data 411, 412 and 413, along with intraoperative data 422 and 424 as well as dimensionality-reduced intraoperative data 451 and 453, and along further with post-operative data 431 and 432, are combined to form a single recording of data for a prior surgery of same nature as the surgical operation under planning, and of an associated prior patient.

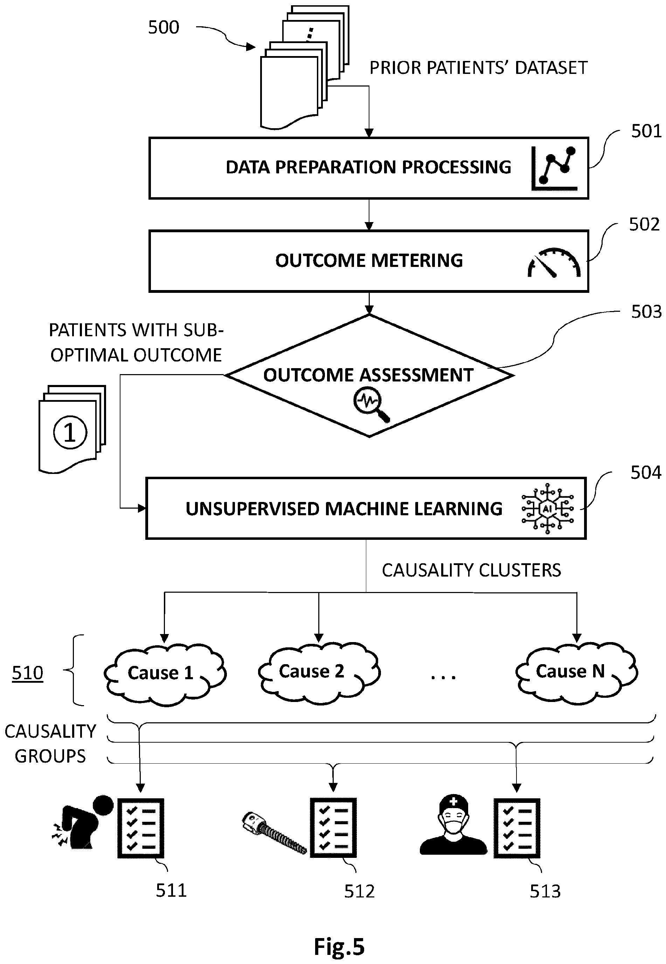

There is shown in **Figure 5** a step diagram illustrating steps of a method of training a predictive algorithm as per embodiments, based on an unsupervised learning process. Said algorithm may be a multivariable linear regression type algorithm.

In step 501, a dataset 50 of data records respectively associated with one prior patient and corresponding prior surgery is received and pre-processed for preparing data. Such data preparation processing 501 may include, for instance, normalization, categorical encoding, word embeddings for natural language transcoding, etc. These exemplary types of pre-processing are non-limitative and may be replaced or mixed with any other suitable pre-processing which may be required by data encoding and/or considered processing. By this pre-processing, the raw data contained in dataset 500 can be converted into a tidy dataset of numeric type, that can then be used for analysis.

In step 502 outcome metering of the pre-processed dataset 500 is performed. In this step, the prior surgeries outcomes reflected by dataset 500 is gauged based on one or more metrics such as threshold-based (specific outcome below a certain value), a percentile (xx %) of the worst performing patients, a given number of patients away from standard deviation from mean of outcomes, missed workdays, etc. Stated otherwise, "outcome metering" is a process aimed at and adapted for quantifying prior patients' surgical benefits (or lack thereof) on the basis of dataset 500.

In step 503, the metered outcome can then be assessed to provide interpretation of prior patients' dataset 500. Analysis of dataset 500 can thus consist in finding the average surgical outcomes of the total listings in the raw data and separating said outcomes into at least two groups. According to embodiments, indeed, interpretation of patients' dataset associated to prior surgeries that has been done in the past includes analysing the pre-processed and metered dataset 500 to find out the average surgical outcomes from the total listings in the raw data. It then further includes grouping the data records 400 of the entire dataset 500 accordingly, for instance in at least two groups of prior patients:
- First group: patients having a rating less than average surgical outcomes (shown as group ① in Figure 5); whereas,
- Second group: patients with ratings higher than average surgical outcomes and which are not immediately interesting.

At step 504, the group ① of data records which correspond to suboptimal surgical outcomes (let us call it data subset ① are used for conducting unsupervised Machine Learning (ML) of the predictive algorithm. This is performed using a ML algorithm configured to analyse and cluster unlabelled records of said data subset ①. These ML algorithms discover hidden patterns or data groupings without the need for human intervention. The predictive algorithm may be any multivariable linear regression type algorithm extended to intraoperative data including, *inter alia,* the intraoperative data 451 and 453 with reduced dimensionality which are based on intraoperative measurements of position and/or orientation of the operated anatomical element, and on intraoperative measurements of the trajectories of at least one surgical instrument during one step of the surgery.

In some embodiments, the ML algorithm provides unsupervised clustering, to identify a given number N of compounded respective causes 510 of suboptimal outcomes, *e.g.,* Cause 1, Cause 2, ..., Cause N, where N is an integer greater than 1.

Cluster analysis is an unsupervised machine learning task that involves automatically discovering natural groupings in data. More precisely, clustering algorithms simply interpret the input data and find natural groups or clusters in the space of characteristics. As such, cluster analysis is an iterative process in which subjective evaluation of the identified clusters is used to modify the algorithm configuration until a desired or appropriate outcome is obtained. It would go far beyond the aims of this description to attempt to detail how this iterative process works on the learning data in question. Instead, reference will be made to existing unsupervised learning models.

Currently popular algorithms are, for instance:
- *K-Means* which may be the most widely known clustering algorithm and involves assigning datasets to clusters in an effort to minimize the variance within each cluster: datasets can be divided into a specific number of clusters by minimizing the total squared distances between the data points and the centers of each cluster;
- *Mini-Batch K-Means* which is a modified version of K-means that makes updates to the cluster centroids using mini-batches of samples rather than the entire dataset;
- *DBSCAN* (standing for "Density-Based Spatial Clustering of Applications with Noise"), which involves finding high-density areas in the domain and expanding those areas of the feature space around them as clusters;
- *SOM* (standing for "Self-Organizing Map") or *SOFM* (standing for "Self-Organizing Feature Map"), which is is an unsupervised machine learning technique used to produce a low-dimensional (typically two-dimensional) representation of a higher dimensional data set while preserving the topological structure of the data; and,
- *Gaussian Mixture,* summarizes a multivariate probability density function with a mixture of Gaussian probability distributions as its name suggests.

Further examples of clustering algorithms include *Mean Shift, Affinity Propagation, Agglomerative Clustering, BIRCH* (standing for "Balanced Iterative Reducing and Clustering using Hierarchies"), *OPTICS* (standing for "Ordering Points To Identify the Clustering Structure"), Spectral Clustering, etc.

The person with ordinary skills in the field, based on general knowledge of machine learning and in view of the specific teachings and guidance provided herein, will be able to identify the unsupervised learning algorithm(s) that can be used, based on criteria such as the volume of data supported, the number of features and attributes that are available, etc. The implementation of such an algorithm based on learning data as proposed in the embodiments taught herein, enables the discovery of hidden patterns in data associated with prior surgeries of the same nature as the surgical operation that is being planned.

In the example as shown in Figure 5, the causes of suboptimal outcomes, from Cause 1 to Cause N, are then grouped in, *e.g.,* three groups of causes, namely:
- causes 511 linked to the associated prior patient;
- causes 512 linked to (at least one) implant used during the surgery; and,
- causes 513 linked to the surgeon having operated during said surgery.

As the skilled person will appreciate, groups are defined by interpreting the clustering results. Some groups can be easier to identify than others. In some embodiments, such grouping can be based on the identification of those metrics that deviate from corresponding mean values. This is an example only, and other groups formed by the automatic clustering may retain attention and be taken into consideration by the surgeon when planning the surgical operation of the patient of interest. Further, and although completely separate groups of causes would seem to be of most use, there is nothing to prevent a given cause for suboptimal outcome belonging to more than one group of cause, showing that a combination of causes rather than a single cause, could be the root cause of the poor outcomes. This is of particular interest in the present invention, as the relatively large amount of data available precludes a conventional statistical analysis.

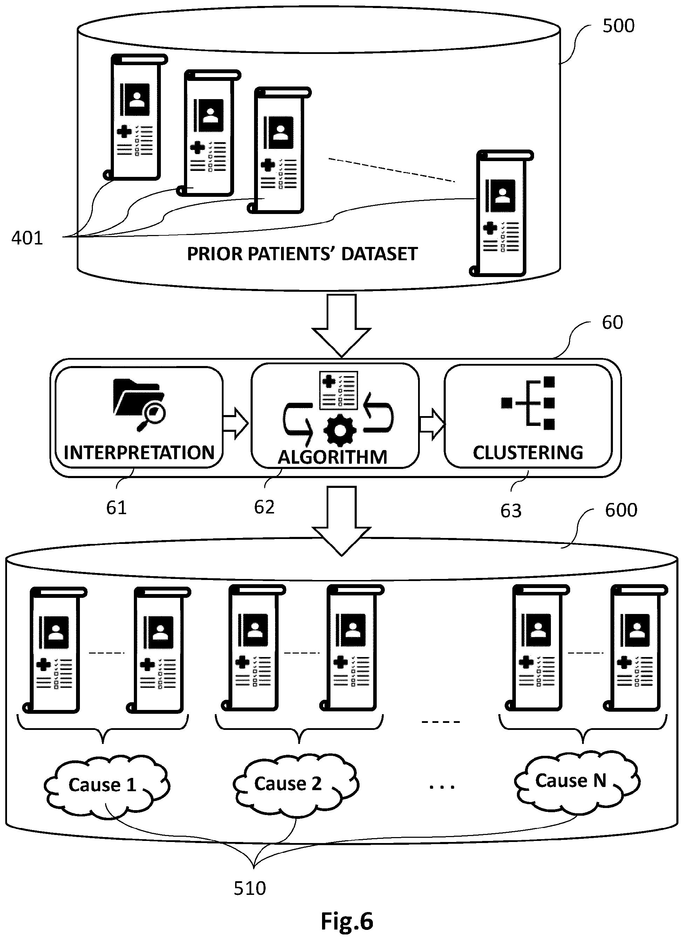

**Figure 6** schematically illustrates the principle of machine learning scheme which is implemented, in a learning phase of the method according to embodiments, by software, in a software unit 60 adapted for configuring the predictive algorithm based on the learning data in the input dataset 500.

The input dataset 500 is comprised of a set of prior patient data 401, generated as described in the foregoing with reference, in particular, to Figure 4. Given that this dataset is relatively large, whereby its manual labelling would be tedious and/or impractical, embodiments preferably use unsupervised machine learning, which consists in clustering the data. According to unsupervised learning schemes, indeed, the predictive algorithm 62 implemented in the software unit 60 learns from unlabelled learning data without predefined results or target variables.

Unlike supervised learning (like predictive modelling) which requires feeding the algorithm a large amount of data and requires data scientists to train the algorithm how to output the correct result for a given input dataset, unsupervised learning has no "training" phase. Thus, the learning data does not need to be manually curated nor to be labelled according to a defined set of input variables and categorized into a defined set of output possibilities prior to being fed into the algorithm under training. Instead, the algorithm is simply provided in input a dataset and uses the variables within the data to identify and separate out natural clusters. This is named "cluster analysis" or "clustering".

With reference to Figure 6, the software unit 60 implement the predictive algorithm 62, as well as an interpretation engine 61 and a clustering engine 63. Said interpretation engine 61 is configured to carry out steps 501, 502 and 503 of the method illustrated by the step diagram of Figure 5, whereas said clustering engine 63 is configured to implement the unsupervised machine learning step 504 of said figure. The machine learning step 504 can be iterated until it identifies interrelationships between the input data 401, thereby sorting these data into clusters, based on the causality of the suboptimal outcomes reflected in the input dataset 500.

In some embodiments, unsupervised learning is applied to perform patient segmentation. Advantageously, the algorithm is naturally blind to inherent biases in how doctors may look at their own patient demographics and only looks at the data for the purpose of achieving clustering. The result is that unsupervised learning can output a unique set 600 of patient segments for whom prior surgery has revealed to be sub-optimal, with implications for the planning (and, possibly also execution) by the surgeon of future surgical operations of same or similar nature to be practiced on new patients.

It would be beyond the purpose of the present description to provide a detailed description of how such algorithms are to be implemented, and the skilled person will be able to choose and put into practice one of these or still other algorithms based on the nature and content definition of the training dataset as disclosed herein. While these algorithms are complex, medical care centers like hospitals do not need an in-house team of data scientists to implement them. Users can rely on automation services readily available from data-driven companies.

Once trained, the algorithm 62 is then able to provide relevant predictions when used with new patient data during a production phase of the proposed method.

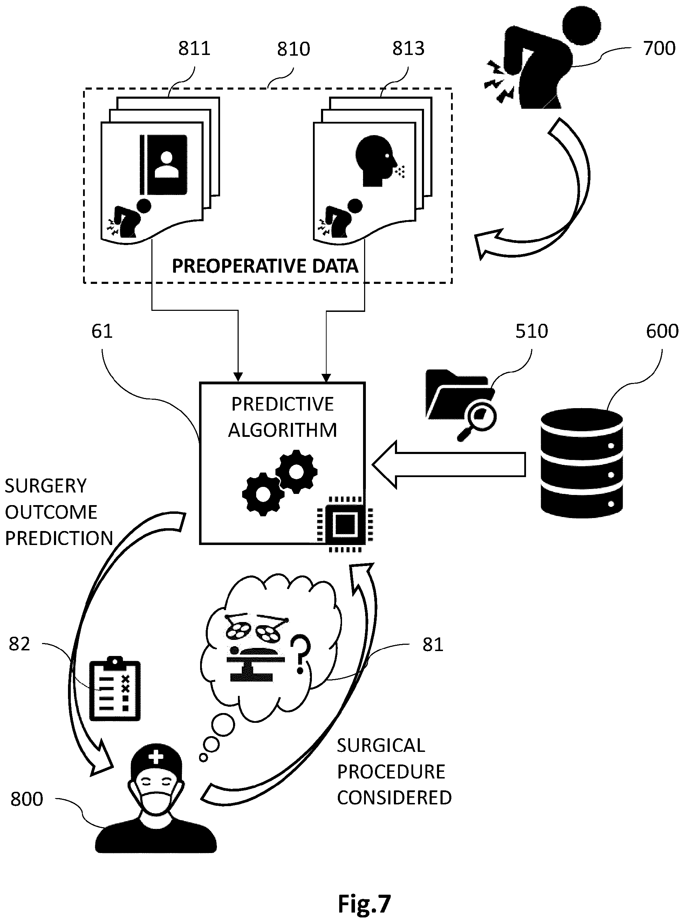

With reference to **Figure 7****,** indeed, the predictive algorithm 61 can be computed, in a so-called production phase, for generating predictions 82 of surgical outcomes for a surgical operation under planning, based on preoperative data associated to said surgical operation, in view of the clustered data 510 included in the set 600 of patient segments for whom prior surgery has revealed to be sub-optimal.

According to embodiments, the afore-mentioned preoperative data comprises preoperative data 810 associated with the patient of interest 700, namely the patient for whom said surgical operation is planned. In addition, the preoperative data also includes preoperative data 81 relating to a surgical procedure being considered by the surgeon 800 for the surgical operation under planning.

For example, preoperative data 810 associated with the patient of interest 700 may include clinical data 811 of the new patient 700, preoperative data 813 representative of Patient Reported Outcomes (PRO). These preoperative data 811 and 813 of new patient 700 are similar to preoperative data 411 and 413, respectively, associated to prior patients 400, which have been presented in the foregoing with reference to Figure 4.

Further, preoperative data 81 relating to a surgical procedure considered for the surgical operation being planned may include, in a non-limiting manner, one or more of the following:
- the characteristics of at least one implantable device whose implantation is envisaged for the planned surgical operation;
- data relating to at least one implantable substance whose implantation is envisaged for the planned surgical operation; and,
- data relating to a surgical approach technique whose use is contemplated to insert the above implantable device and/or the above implantable substance during the surgical operation at stake.

In some embodiments, the generated predictions 82 can include a metric of the risk of obtaining for said surgical operation under planning one or more surgical outcomes lower than standard surgical outcomes obtained for at least some of the prior surgeries. Such sub-optimal outcomes may represent, for instance, a loss of correction of the patient's intervertebral spacing for spinal surgery. Said metric may have one or more components, respectively associated to different kind of outcomes, namely outcomes tied to respective criteria which may be appreciated for evaluating the surgery outcome.

Thanks to the predictions 82 of surgical outcomes that can be expected from any surgical protocol envisaged to operate on the patient concerned, the surgeon 800 is thus provided with useful information for selecting, adapting and/or refining the details of the surgical procedure under consideration. This occurs preoperatively during the planning stage.

Furthermore, while performing the operation in question, and in the light of any additional information revealed and/or potentially derived from the performance of the operation in progress, the surgeon can use updated predictions produced by the device according to the embodiments of the invention to make more appropriate decisions intraoperatively. For instance, the surgeon may decide to deviate from initially established plan and, to do so, may rely on updated predictions of the outcomes that are expected to result from the envisaged modification of the surgical procedure.

The present invention can also be embedded in a computer program product, which comprises all the features enabling the implementation of the methods described herein, and which - when loaded in an information processing system - is able to carry out these methods. Computer program means or computer program in the present context mean any expression, in any language, code or notation, of a set of instructions intended to cause a system having an information processing capability to perform a particular function either directly or after conversion to another language. Such a computer program can be stored on a computer or machine readable medium allowing data, instructions, messages or message packets, and other machine readable information to be read from the medium. The computer or machine readable medium may include non-volatile memory, such as ROM, Flash memory, Disk drive memory, CD-ROM, and other permanent storage. Additionally, a computer or machine readable medium may include, for example, volatile storage such as RAM, buffers, cache memory, and network circuits. Furthermore, the computer or machine readable medium may comprise computer or machine readable information in a transitory state medium such as a network link and/or a network interface, including a wired network or a wireless network, that allow a device to read such computer or machine readable information.

Examples that were indicated as such in the description which precedes are purely illustrative and are not meant to be restrictive of the invention. Expressions such as "comprise", "include", "incorporate", "contain", "is" and "have" are to be construed in a non-exclusive manner when interpreting the description and its associated claims, namely construed to allow for other items or components which are not explicitly defined also to be present. Reference to the singular is also to be construed in be a reference to the plural, and vice versa.

While there has been illustrated and described what are presently considered to be the preferred embodiments of the present invention, it will be understood by those skilled in the art that various other modifications may be made, and equivalents may be substituted, without departing from the true scope of the present invention. Additionally, many modifications may be made to adapt a particular situation to the teachings of the present invention without departing from the central inventive concept described herein. Furthermore, an embodiment of the present invention may not include all of the features described above. Therefore, it is intended that the present invention not be limited to the particular embodiments disclosed, but that the invention include all embodiments falling within the scope of the appended claims.

In the above description, well-known functions or constructions by a person skilled in the art have not been described in detail since they would obscure the invention in unnecessary detail.

The one skilled in the art will readily appreciate that various parameters disclosed in the description may be modified and that various embodiments disclosed and/or claimed may be combined without departing from the scope of the invention.

It is stipulated that the reference signs in the claims do not limit the scope of the claims, but are merely inserted to enhance the legibility of said claims.

## Claims

1. A device for assisting a surgeon of interest (800) in a preoperative planning and/or in the execution of a surgical operation on a given anatomical site (3) of a patient of interest (700), comprising a software unit (60) adapted to produce, during a planning stage, predictions (82) of surgical outcomes for the surgical operation under consideration, based on a predictive algorithm (61) using preoperative data (810, 81) associated with the surgical operation under consideration, wherein:
- the software unit (60) is configured to be trained using learning data (400) which comprise preoperative data (410), intraoperative data (420) and postoperative data (430) collected for a determined patient population during prior surgeries of same nature as said surgical operation under consideration which have been performed at the same anatomical site on respective prior patients of said patient population with the assistance of a localization system, of which, respectively:
-- the preoperative data (410) comprise, for each prior patient of said determined patient population:
--- data relating to a clinical profile of said prior patient; and,
--- data relating to a surgical protocol that was implemented during the prior surgery on said prior patient;
-- the intraoperative data (420) comprise recordings representative of trajectories as a function of time of the position and/or orientation during the prior surgery of at least one anatomical element (31), relative to said prior patient's anatomical site (3); and,
-- the postoperative data (430) comprise data representative of surgical outcomes of the prior surgeries for each prior patient of the determined patient population,
- the software unit is configured to produce predictions (82) of surgical outcomes for the surgical operation under consideration based on the preoperative data (810,81) associated with said surgical operation under consideration, which comprises:
-- data relating to the clinical profile (811) and/or patient reported outcomes (813) of the patient of interest (700); and,
-- data relating to a surgical protocol (81) being considered by the surgeon of interest (800) for the surgical operation under consideration.

2. Device according to claim 1, wherein the recordings representative of trajectories as a function of time of the position and/or orientation of the associated prior patient's operated anatomical element comprise time-dependent measurements of the intraoperative variation in position and/or orientation, respectively, of said prior patient's operated anatomical element relative to said prior patient's anatomical site.

3. Device according to claim 2, wherein the intraoperative variation in position and orientation of the associated prior patient's operated anatomical element is determined by translation values and rotation values, respectively, of said prior patient's operated anatomical element, between the beginning and the end of the prior surgery, said values being defined in a referential of the prior patient's anatomical site.

4. Device according to any one of claims 1 to 3, wherein the intraoperative data associated with the prior surgery (420) further comprise position values and/or orientation values (422), with respect to the associated prior patient's operated anatomical element, of an implant implanted during the associated prior surgery.

5. Device according to claim 4, wherein the position values and/or orientation values (422) of the implant with respect to the associated prior patient's operated anatomical element are derived from position values and/or orientation values, respectively, of at least one surgical instrument equipped with a position and/or orientation tracker of the localization system and carrying the implant at the time of placement and deposition of said implant in the prior patient's anatomical site.

6. Device according to any one of claims 1 to 5, wherein the intraoperative data associated with the prior surgery further comprise time-dependent records (423) representative of the use, within the associated prior patient's anatomical site, during the associated prior surgery of at least one surgical instrument (70) used by the surgeon of interest during at least one specific surgical step of said prior surgery.

7. Device according to any one of claims 1 to 6, wherein the predictions (82) of surgical outcomes of the surgical operation under consideration include a metric of the risk of obtaining for said surgical operation under consideration one or more surgical outcomes inferior to an average surgical outcomes.

8. Device according to any one of claims 1 to 7, wherein preoperative data relating to a surgical protocol (412) that was planned and has been implemented during a prior surgery and/or preoperative data relating to a surgical protocol (81) considered for the surgical operation under consideration include one or more of:
- characteristics of at least one implantable device (38), that has been implanted during said prior surgery or implantation of which is considered for the surgical operation under consideration, respectively;
- data relating to at least one implantable substance which has been implanted during said prior surgery or whose implantation is being considered for the surgical operation under consideration, respectively; and,
- data relating to a surgical approach technique used during said prior surgery to insert said implantable device (38) and/or said implantable substance, or the use of which is being considered for the surgical operation under consideration, respectively.

9. Device according to any one of claims 1 to 8, wherein data relating to a clinical profile (411) of a prior patient (400) and/or data relating to the clinical profile (811) of the patient of interest (700) comprise categorical variables corresponding to personal information pertaining to said prior patient (400) or to said patient of interest (700), respectively, said personal information including at least one of gender, ethnicity, anatomical level of operated vertebrae within the patient's spine, frontal and/or profile X-ray images, Roussouly or Lenke classification, spino-pelvic configuration, patient score on a health questionnaire.

10. Device according to any one of claims 1 to 9, wherein the postoperative data (430) representative of the surgical outcomes of a prior surgery comprise:
- outcomes (431) reported by a prior patient (400) and collected after the prior surgery on said prior patient; and/or
- one or more measurements (432) performed on medical imaging pictures of the prior patient's anatomical site obtained after said prior surgery on said prior patient.

11. Device according to any one of claims 1 to 10, wherein the software unit (60) comprises learning data pre-processing module (441,443) for reducing dimensionality of:
- recordings (421) representative of the trajectories as a function of time of the position and/or orientation of the prior patient's anatomical element operated on; and/or
- recordings (423) representative of the use of the surgical instrument (70) as claimed in claim 6.

12. Device according to claim 11, wherein the means (441,443) for reducing dimensionality comprise a pre-trained encoder and/or a Principal Component Analysis (PCA) algorithm.

13. Device according to any one of claims 1 to 12, wherein the prior surgeries are surgeries performed specifically by the surgeon of interest, or by a college of surgeons including said surgeon of interest.

14. Device according to any one of claims 1 to 13, wherein the predictive algorithm (61) is a multivariable algorithm.

15. Device according to Claim 14, wherein the predictive algorithm (61) is a multivariable linear regression type algorithm.

16. Method for assisting a surgeon of interest (800) in a preoperative planning and/or in the execution of a surgical operation on a given anatomical site (3) of a patient of interest (700), the method being implemented by a software unit and comprising the production, during a planning stage, of predictions (82) of surgical outcomes for the surgical operation under consideration, based on a predictive algorithm (61) using preoperative data (810,81) associated with said surgical operation under consideration, wherein:
- in an unsupervised learning phase, the software unit is provided with an input dataset (500) comprising learning data which includes preoperative data (410), intraoperative data (420) and postoperative data (430) associated with a plurality of prior surgeries of same nature as the surgical operation under consideration which have been performed at the same anatomical site on respective prior patients (400) with the assistance of a localization system, of which, respectively:
-- the preoperative data comprise:
--- data relating to a clinical profile (411) and/or patient reported outcomes (413) of the associated prior patient (400); and,
--- data (412) relating to a surgical protocol that was planned and implemented during the associated prior surgery;
-- the intraoperative data comprises recordings representative of trajectories as a function of time of the position and/or orientation during the prior surgery of at least one anatomical element operated on at the associated prior patient's anatomical site (3), relative to said prior patient's anatomical site; and,
-- the postoperative data (430) comprises data representative of surgical outcomes of the associated prior surgery,
- in a production phase, the software unit produces at least one prediction (82) of surgical outcomes for the surgical operation under consideration based on the preoperative data (810) associated with said surgical operation under consideration, which comprises:
-- data relating to the clinical profile (811) and/or patient reported outcomes (813) of the patient of interest (700); and,
-- data relating to a surgical protocol (81) being considered for the surgical operation under consideration.

17. A computer program product comprising one or more stored sequences of instructions that are accessible to a processor and which, when executed by the processor, cause the processor to carry out all the steps of the method of claim 16.
